# EUROPEAN PATENT APPLICATION

(11) **EP 4 545 071 A1**
(43) Date of publication of application: **30.04.2025**
(21) Application number: 23827572.1
(22) Date of filing: 23.06.2023
(51) Int. Cl.: A61K 9/16, A61K 47/12, A61K 9/00

(54) **SUSTAINED-RELEASE MICROPARTICLES CONTAINING DRUG AND PAMOIC ACID**

(30) Priority: 23.06.2022 KR 20220077134
(71) Applicant: G2GBIO, Inc., Cheongju-si, Chungcheongbuk-do 28161 (KR)
(72) Inventor: KIM, Hyemin, Cheongju-si, Chungcheongbuk-do 28222 (KR); KIM, Boyeon, Cheongju-si, Chungcheongbuk-do 28222 (KR); LEE, Yeonkyeong, Sejong 30150 (KR); PARK, Donghyun, Daejeon 34053 (KR); LEE, Jinwoo, Sejong 30144 (KR); WON, Dongpil, Daejeon 34007 (KR); SEOL, Eunyoung, Daejeon 34080 (KR); LEE, Heeyong, Daejeon 34032 (KR)
(74) Representative: Ricker, Mathias
(86) International application number: PCT/KR2023/008797
(87) International publication number: WO 2023/249464

(57) **Abstract**

A sustained-release microsphere containing a drug, pamoic acid, and a biocompatible polymer, a pharmaceutical composition containing the sustained-release microsphere for prevention, improvement, or treatment of hepatitis B, prostate cancer, breast cancer, endometriosis, uterine fibroid, precocious puberty, acromegaly, gastro·entero·pancreatic endocrine tumor, Alzheimer's disease, or cognitive disorder, and a preparing method of the sustained-release microsphere are provided.

## Description

### TECHNICAL FIELD

The present disclosure relates to a sustained-release microsphere containing a drug and pamoic acid, and more specifically, to a sustained-release microsphere formulation containing a drug and pamoic acid, which encapsulates a high content of drug and exhibits stable drug release characteristics for long period, and a method of preparing the same.

### BACKGROUND

A microsphere formulation using a biocompatible polymer in order to develop a sustained-release formulation has developed into an active area of research interest and clinical application. However, even though a microsphere using only the biocompatible polymer is not a nanoparticle, the microsphere exhibits an "initial burst" phenomenon in which a rapid drug release occurs at a beginning of the release. Such an "initial burst" phenomenon may cause side effects due to a rapid increase in drug concentration, and there is a problem that the drug rapidly released in an initial stage is metabolized without exhibiting medical effects.

As a result of extensive research to solve the problem of rapid initial release of the drug in such a microsphere, the inventors of the present disclosure have confirmed that pamoic acid may be used as a pharmaceutically usable salt or additive that may act as a release-controlling agent for the drug in the microsphere. Furthermore, through repeated research, the inventors of the present disclosure have confirmed an unexpected problem in which a degree of release varies depending on an amount of sodium contained in the pamoic acid.

Accordingly, the inventors of the present disclosure have completed the development of a sustained-release microsphere injectable solution having the characteristic of stable drug release for long period of time of encapsulating a high content of the drug while solving problems of the related art.

### SUMMARY

### TECHNICAL PROBLEM

The present disclosure is devised to solve the problems of the related art as described above, and provides a sustained-release microsphere formulation which encapsulates a high content of drug and exhibits stable drug release characteristics for a long period, and a method of preparing the same.

In addition, the present disclosure uses pamoic acid as a pharmaceutically usable salt or an additive for solving excessive initial release of a high-content drug, and particularly, provides a method of preparing a sustained-release injection having a long-term efficacy by quantitatively controlling sodium or potassium contained in the pamoic acid.

### TECHNICAL SOLUTION

In an aspect of the present disclosure, it is possible to provide a sustained-release microsphere containing a drug, pamoic acid, and a biocompatible polymer in which a content of sodium contained in the microsphere is less than 1,600 ppm.

In an aspect of the present disclosure, it is possible to provide a sustained-release microsphere prepared using a drug, pamoic acid, and a biocompatible polymer in which a molar ratio of the drug to the pamoic acid is 1:0.01 to 1:1, and the content of sodium contained in a microsphere is less than 1,600 ppm.

In an aspect of the present disclosure, it is possible to provide a sustained-release microsphere prepared using a drug, pamoic acid, and a biocompatible polymer in which a molar ratio of the drug to the pamoic acid is 1:0.01 to 1:1, and a content of sodium contained in the pamoic acid is 10 wt% or less based on the total weight of the pamoic acid.

In an aspect of the present disclosure, the sustained-release microsphere may further contain a pamoate of a drug.

In an aspect of the present disclosure, the drug may be one or more selected from the group consisting of donepezil, rivastigmine, entecavir, leuprorelin, octreotide, memantine, lamivudine, rotigotine, ropinirole, bupivacaine, ropivacaine, meloxicam, buprenorphine, fentanyl, nimodipine, granisetron, triamcinolone, cytarabine, carmustine, tamsulosin, polmacoxib, testosterone, estradiol, risperidone, paliperidone, olanzapine, aripiprazole, goserelin, triptorelin, buserelin, nafarelin, deslorelin, pasireotide, lanreotide, vapreotide, exenatide, liraglutide, lixisenatide, semaglutide, or pharmaceutically acceptable salts thereof.

In an aspect of the present disclosure, the initial release rate on day 1 of the drug may be less than 15%.

In an aspect of the present disclosure, a content of the drug may be 10 to 79 wt% based on the total weight of the sustained-release microsphere.

In an aspect of the present disclosure, the biocompatible polymer may be 20 to 85 wt% based on the total weight of the sustained-release microsphere.

In an aspect of the present disclosure, the pamoic acid may be 1 to 30 wt% based on the total weight of the sustained-release microsphere.

In an aspect of the present disclosure, the drug in the sustained-release microsphere may be released for 1 month to 6 months.

In an aspect of the present disclosure, the microsphere may be for injection.

In an aspect of the present disclosure, it is possible to provide a pharmaceutical composition containing a sustained-release microsphere for prevention, improvement, or treatment of hepatitis B, prostate cancer, breast cancer, endometriosis, uterine fibroid, precocious puberty, acromegaly, gastro·entero·pancreatic endocrine tumor, Alzheimer's disease, or cognitive disorder.

In an aspect of the present disclosure, it is possible to provide a preparing method of the sustained-release microsphere.

### ADVANTAGEOUS EFFECTS

A sustained-release microsphere formulation of the present disclosure can increase patient compliance, thereby maximize therapeutic efficacy by maintaining a therapeutic concentration range of a drug for a long period without excessive initial release of the drug.

### BRIEF DESCRIPTION OF THE DRAWINGS

FIG. 1 is a graph showing an *in-vitro* drug release rate on day 1 depending on the content of sodium in the sustained-release microsphere containing donepezil.
FIG. 2 is a graph showing an *in-vivo* drug release rate depending on the content of sodium in pamoic acid in the sustained-release microsphere containing donepezil.

### BEST MODE

Hereinafter, the present disclosure will be described in more detail.

In an embodiment, the present disclosure relates to a sustained-release microsphere containing a drug, pamoic acid, and a biocompatible polymer in which a content of sodium in a microsphere is less than 1,600 ppm.

In another embodiment, the present disclosure relates to a sustained-release microsphere prepared using a drug, pamoic acid, and a biocompatible polymer in which a molar ratio of the drug to the pamoic acid is 1:0.01 to 1:1, and a content of sodium in a microsphere is less than 1,600 ppm.

In another embodiment, the present disclosure relates to a sustained-release microsphere prepared using a drug, pamoic acid, and a biocompatible polymer in which a molar ratio of the drug to the pamoic acid is 1:0.01 to 1:1, and a content of sodium contained in the pamoic acid is 10 wt% or less based on the weight of the pamoic acid.

In another embodiment, the present disclosure relates to a sustained-release microsphere containing a drug, pamoic acid, and a biocompatible polymer in which potassium is contained in the microsphere.

In still another embodiment, the present disclosure relates to a sustained-release microsphere prepared using a drug, pamoic acid, and a biocompatible polymer in which a molar ratio of the drug to the pamoic acid is 1:0.01 to 1:1, and potassium is contained in the pamoic acid.

A microsphere formulation using a biocompatible polymer in order to develop a conventional sustained-release formulation has developed into an active area of research interest and clinical application. However, in order to prepare such a microsphere formulation for a long-term continuous administration, a drug contained in the microsphere should be contained in a significantly high amount, by considering an administration period and dosage. In such a case where the drug is contained in the microsphere at a high content, even when a biocompatible polymer having a sufficiently long decomposition rate is applied, a desired administration period may not be met because a release period is not be sufficiently long due to the high content of the drug or an "initial burst" problem in which the drug is released too quickly may occur at a beginning of the release. In this case, various side effects may occur due to a rapid increase in blood concentration of the drug, and in particular, serious problems may occur in a case of a drug having a low therapeutic index. Meanwhile, if the content of the drug contained in the microsphere is lowered to solve these problems, there is a problem that single dose of the microspheres is excessively increased because of insufficient drug content for treatment, thereby causing incompliance and discomfort of a patient.

Accordingly, the present inventors found that the addition of pamoic acid together with a drug for the preparation of the microsphere, could solve the problems of initial rapid release and the drug release not occurring over a sufficient period of time. even if a large amount of drug was contained. However, the present inventors also found that when pamoic acid was used for the preparation of the microsphere, the release of the drug in the microsphere was significantly affected unexpectedly by sodium or potassium content contained in the pamoic acid.

Accordingly, the present disclosure provides a microsphere containing a drug as an active ingredient, pamoic acid, and a biocompatible polymer, wherein the content of sodium in the microsphere is less than 1,600 ppm. Preferably, the content of sodium may be less than 1,550 ppm, less than 1,500 ppm, less than 1,450 ppm, less than 1,400 ppm, less than 1,350 ppm, less than 1,300 ppm, less than 1,250 ppm, less than 1,200 ppm, less than 1,150 ppm, less than 1,100 ppm, less than 1,050 ppm, or less than 1,000 ppm. Alternatively, the content of sodium may be 0.01 to 1,600 ppm, 0.1 to 1,400 ppm, 1 to 1,200 ppm, 10 to 1,000 ppm, or 100 to 900 ppm.

Specifically, any drug may be used as a drug contained as an active ingredient in the microsphere of the present disclosure as long as it is intended for sustained or controlled release for a desired period of time. Examples of such drug may contain at least one selected from the group consisting of donepezil, rivastigmine, entecavir, leuprolide, octreotide, and pharmaceutically acceptable salts thereof, but are not limited thereto.

The biocompatible polymer may be at least one selected from the group consisting of poly(lactide-co-glycolide), poly(lactide-co-glycolide)glucose, polylactide, polyglycolide, polycaprolactone, and mixtures thereof; and polyglycolide, polylactide and a copolymer of polyglycolide and polylactide, but is not limited thereto. In a preferred embodiment, a molar ratio of lactide to glycolide in the copolymer may be 40:60 to 90:10, 45:55 to 85:15 or 50:50 to 75:25, for example 45:55, 50:50, 75:25, or 85:15 in a case of polyglycolide and polylactide copolymer.

The biocompatible polymer may have a weight average molecular weight of 4,000 to 240,000. For example, the weight average molecular weight of the biocompatible polymer includes all lower numerical ranges within the above ranges, such as a weight average molecular weight of 4,000 to 100,000, a weight average molecular weight of 7,000 to 50,000, a weight average molecular weight of 5,000 to 20,000, a weight average molecular weight of 10,000 to 18,000, and a weight average molecular weight of 18,000 to 28,000.

When two or more types of the biocompatible polymers are contained, the biocompatible polymer may be a combination or blend of polymers that the types of the exemplified polymers are different from each other, but may be a combination of polymers in which the same types of polymers have different intrinsic viscosities and/or monomer ratios (for example, a combination or blend of two or more poly(lactide-co-glycolide) having different intrinsic viscosities), or polymer having different terminal groups (for example, an ester terminal group or an acid terminal group). Examples of commercially available biocompatible polymers that may be used in the present disclosure may include RG 502H, RG 503H, RG 504H, RG 502, RG 503, RG 504, RG 653H, RG 752H, RG 753H, RG 752S, RG 755S, RG 756S, RG 858S, R 202H, R 203H, R 205H, R 202S, R 203S, R 205S which are the Resomer series from Evonik Rohm GmbH and PDL 02A, PDL 02, PDL 04, PDL 05, PDLG 7502A, PDLG 7502, PDLG 7507, PDLG 5002A, PDLG 5002, PDLG 5004A, PDLG 5004, PDLG 5010, PL 10, PL 18, PL 24, PL 32, PL 38, PDL 20, PDL 45, PC 02, PC 04, PC 12, PC 17, and PC 24 which are manufactured by Corbion alone, or in combination, or in blends, but are not limited thereto. In a preferred embodiment, an intrinsic viscosity of the biocompatible polymer may be 0.16-1.9 dL/g. The intrinsic viscosity of the biocompatible polymer used in the present disclosure is measured at a concentration of 0.1% (w/v) in chloroform at 25°C using an Ubbelohde viscometer.

The sustained-release microspheres may further contain a pamoate of the drug.

In an embodiment, a form of the pamoate of the drug, and/or pamoic acid may be present together with the drug in the sustained-release microsphere of the present disclosure.

In an embodiment, the sodium may be in a form of a monosodium or a disodium.

In addition, a content ratio of the drug to the pamoic acid is 1:0.01 to 1:1 in terms of the molar ratio of a drug free base to the pamoic acid in the microsphere of the present disclosure.

When the above range is satisfied, it has an advantage of being able to maintain an appropriate release rate during the release period without rapid or excessive delay in the release rate of the drug.

It is preferable for the microspheres of the present disclosure to have a uniform particle size distribution. The microspheres having a uniform particle size distribution can be administered in a more accurate amount with less variation during injection than the microspheres having a non-uniform particle size distribution. Preferably, the span value of the microsphere of the present disclosure is 1.2 or less. The term "span value" used herein is an index indicating the uniformity of particle size of the microspheres, and refers to a value obtained by a formula of size distribution (Span value)=(Dv0.9-Dv0.1)/Dv0.5. Here, Dv0.1 refers to a particle size corresponding to 10% of a volume% in the particle size distribution curve of the microspheres, Dv0.5 refers to a particle size corresponding to 50% of the volume% in the particle size distribution curve of the microspheres, and Dv0.9 refers to a particle size corresponding to 90% of the volume% in the particle size distribution curve of the microspheres.

According to the present disclosure, the initial release rate on day 1 of the drug in the microsphere may be less than 15%. Preferably, the initial release rate may be less than 14%, less than 13%, less than 12%, less than 11%, or less than 10%, but is not limited thereto.

In a specific embodiment, the content of the drug may be, 10 to 79 wt%, 10 to 75 wt%, or 10 to 70 wt% based on the total weight of the sustained-release microsphere, but is not limited thereto.

In a specific embodiment, the microsphere of the present disclosure may be a sustained-release microsphere containing donepezil in which a mole ratio of donepezil to pamoic acid is 1:0.05 to 1:1 and the content of which is 40 to 70% (w/w).

In another specific embodiment, the donepezil microsphere (the microsphere containing donepezil) of the present disclosure may be a sustained-release microsphere capable of sustained-release of donepezil for 1 to 6 months in which the initial release on day 1 of the microsphere is 15% or less, 0.01 to 15%, or 0.1 to 15%, and the content of sodium contained in the microsphere is less than 1,600 ppm, or 10 to 1,300 ppm.

In another specific embodiment, the microspheres of the present disclosure may be a sustained-release microsphere capable of sustained-release in which a molar ratio of rivastigmine to pamoic acid is 1:0.3 to 1:1, or 1:0.4 to 1:0.6, rivastigmine which is the active ingredient is contained in an amount of up to 30 wt%, preferably 10 to 30 wt%, or 15 to 26 wt% based on the total weight of the microsphere, the initial release on day 1 is less than 15%, 0.01 to 2.5%, 0.02 to 2.3%, or 0.03 to 2%, and a content of sodium contained in the microsphere is less than 50 ppm, less than 40 ppm, less than 30 ppm, or 5 to 30 ppm.

In another specific embodiment, the microsphere of the present disclosure may be a sustained-release microsphere capable of sustained-release in which a molar ratio of entecavir to pamoic acid is 1:0.0 to 1:0.25, entecavir which is the active ingredient is contained in an amount of 22 wt% or more and 22 wt% to 40 wt% based on the total microsphere, the initial release on day 1 is less than 10%, 0.1 to 5%, or 0.1 to 3%, and a content of sodium in the microsphere is 100 to 300 ppm or less than 150 to 200 ppm.

In another specific embodiment, the microsphere of the present disclosure may be a sustained-release microsphere capable of sustained-release in which a molar ratio of leuprolide to pamoic acid is 1:0.05 to 1:0.1, leuprolide which is the active ingredient is contained in an amount of 10 wt% or more and 10 to 20 wt% or more based on the total microsphere, the initial release on day 1 is 15% or less, and a content of sodium in the microsphere is less than 100 ppm, or 1 to 50 ppm.

In another specific embodiment, the microsphere of the present disclosure may be a sustained-release microsphere capable of sustained-release in which a molar ratio of octreotide to pamoic acid is 1:0.1 to 1:1.2, or 1:0.15 to 1:1.13, octreotide which is the active ingredient is contained in an amount of 10 wt% or more, 10 to 40 wt%, or 10 to 30 wt% based on the total microsphere, the initial release on day 1 is less than 15%, 1 to 12%, or 1 to 10%, and a content of sodium in the microsphere is less than 1,600 ppm.

In a specific embodiment, the drug in the sustained-release microsphere of the present disclosure may be released for 1 month to 6 months, 1 month to 5 months, 1 month to 3 months, or 1 month to 2 months.

In a specific embodiment, the sustained-release microsphere of the present disclosure may be for injection.

In another embodiment, the present disclosure relates to a pharmaceutical composition containing a sustained-release microsphere for prevention, improvement, or treatment of hepatitis B, prostate cancer, breast cancer, endometriosis, uterine fibroid, precocious puberty, acromegaly, gastro·entero·pancreatic endocrine tumor, Alzheimer's disease, or cognitive disorder. More specifically, the pharmaceutical composition may further contain a pharmaceutically acceptable carrier. Preferably, the pharmaceutical composition may be an injectable formulation.

Hereinafter, a preparing method of a sustained-release microsphere injection of the present disclosure will be described in detail.

The sustained-release microsphere of the present disclosure may be prepared, for example, using a "solvent extraction and evaporation method", but is not limited thereto.

As a specific example of the sustained-release microsphere preparing method of the present disclosure, the preparing method of the sustained-release microsphere including the following steps is provided:
(a) preparing a dispersed phase by dissolving a drug; pamoic acid, and a biocompatible polymer in a solvent for the dispersed phase;
(b) preparing an emulsion by adding the dispersed phase prepared in the step (a) to an aqueous solution containing a surfactant as a continuous phase;
(c) preparing a suspension containing the microsphere by extracting the solvent for the dispersed phase of the dispersed phase in the emulsion prepared in the step (b) into a continuous phase, and evaporating the extracted solvent to form a microsphere; and
(d) preparing the microsphere by recovering the microsphere from the suspension of the step (c),
wherein, a content of sodium in the pamoic acid is 10 wt% based on the total weight of the pamoic acid.

In the preparing method, matters relating to the drug, the pamoic acid, the sodium or the potassium and the biocompatible polymer, etc., may be applied as described in the microsphere unless otherwise mentioned.

Specifically, a molar ratio of the drug to the pamoic acid may be 1:0.01 to 1:1 based on the free base form of the active ingredient.

In addition, in a specific embodiment, the drug may be in the free base form in step (a), and the pamoic acid may be contained in a form of a powder or a solution.

In a specific embodiment, the step (a) is performed by adding the pamoic acid to a solution of the drug dissolved in the solvent for the dispersed phase.

The solvent for the dispersed phase is not limited, but may include dichloromethane, ethyl acetate, ethyl formate, methyl acetate, methyl formate, butyl acetate, n-propyl acetate, isopropyl acetate, n-propyl formate, glycofurol, methyl isopropyl ketone, dimethyl carbonate, or mixed solvents thereof.

In a specific embodiment, the solvent for the dispersed phase may be a mixed solvent, and in this case, it is preferable that a solvent having a property of being immiscible with water among the mixed solvent is used in an amount of at least 30% (w/w), 30 to 90% (w/w), 30 to 85% (w/w), 40 to 90% (w/w), 40 to 85% (w/w) based on the total weight of the mixed solvent. When preparing the microsphere of the present disclosure, the lower the content of sodium of the pamoic acid, the more the used amount of the solvent for the dispersed phase may be reduced.

In addition, the solvent for the dispersed phase is used as the first solvent and additionally, may further contain one or more selected from the group consisting of chloroform, acetone, acetonitrile, dimethyl sulfoxide, dimethylformamide, n-methylpyrrolidone, methyl ethyl ketone, acetic acid, methyl alcohol, ethyl alcohol, propyl alcohol, benzyl alcohol, and mixed solvents thereof as the second solvent.

A method of homogeneously mixing the dispersed phase and the continuous phase containing the surfactant in the step (b) is not particularly limited, but may be performed using a high-speed stirrer, an inline mixer, a membrane emulsion method, a microfluidics emulsion method, etc.

The type of the surfactant used in the step (b) is not particularly limited, and any type that may help the dispersed phase to form a stable droplet-shaped dispersed phase in the continuous phase may be used. The surfactant may preferably be selected from the group consisting of methyl cellulose, polyvinylpyrrolidone, carboxymethylcellulose, lecithin, gelatin, polyvinyl alcohol, polyoxyethylene sorbitan fatty acid ester, polyoxyethylene castor oil derivative, and mixtures thereof, and most preferably polyvinyl alcohol may be used as the surfactant.

In the step (b), the content of the surfactant in the continuous phase containing the surfactant may be 0.01 w/v% to 20 w/v%, preferably 0.1 w/v% to 5 w/v%, based on the total volume of the continuous phase containing the surfactant. When the content of surfactant is less than 0.01 w/v%, the droplet-shaped dispersed phase or the emulsion may not be formed in the continuous phase, and when the content of the surfactant exceeds 20 w/v%, it may be difficult to remove the surfactant after fine particles are formed in the continuous phase due to an excessive amount of the surfactant.

Water may be used as the solvent of the continuous phase used in the step (b), and water partially containing one or more selected from the group consisting of methyl alcohol, ethyl alcohol, propyl alcohol, ethyl formate, methyl acetate, methyl formate, butyl acetate, n-propyl acetate, isopropyl acetate, n-propyl formate, glycofurol, methyl isopropyl ketone, dimethyl carbonate, and ethyl acetate may be used in order to control an extraction speed of an organic solvent from the dispersed phase in an emulsion state.

In the step (c), when the emulsion containing the droplet-shaped dispersed phase and the continuous phase containing the surfactant is maintained or stirred at a temperature below a boiling point of the organic solvent for a certain period of time, for example, 2 hours to 48 hours, the organic solvent may be extracted from the droplet-shaped dispersed phase to the continuous phase. Some of the organic solvent extracted to the continuous phase may be evaporated from a surface. As the organic solvent is extracted from the droplet-shaped dispersed phase, the droplet-shaped dispersed phase may be solidified to form the microsphere.

In order to additionally and efficiently remove the organic solvent in the step (c), heat may be applied to the temperature of the continuous phase for a certain period of time.

In the step (d), a method of recovering the microsphere of the present disclosure may be performed using various known techniques, for example, a method such as filtration or centrifugation may be used.

Between the steps (c) and (d), the remaining surfactant may be removed through filtration and washing, and the microsphere may be recovered by filtering again.

The washing step for removing the remaining surfactant may be typically performed using water, and the washing step may be repeated several times.

The preparing method of the present disclosure may obtain a finally dried microsphere by drying the obtained microsphere using a conventional drying method after the step (d) or after the filtering and washing steps.

Hereinafter, preferred examples of the present disclosure will be described in order to facilitate understanding of the present disclosure. It will be apparent to those skilled in the art, however, that the following examples are illustrative of the present disclosure and that various changes and modifications may be made within the scope and spirit of the present disclosure.

### EMBODIMENT OF INVENTION

### [Examples]

Hereinafter, the present disclosure will be described in more detail through examples. These examples are for illustrative purposes only, and the present disclosure is not intended to be limited by these examples.

### Preparation Example 1. Preparation of a sustained-release microsphere containing donepezil

A donepezil microsphere was prepared in order to verify the effect depending on the content of sodium of a sustained-release microsphere containing donepezil of the present disclosure.

A dispersed phase was used after mixing a biocompatible polymer, a donepezil base (manufacturer: Neuland Laboratories, India), and pamoic acid (manufacturer: Kyungbo Pharmaceutical, TCI) with dichloromethane (manufacturer: J.T Baker, USA) or a mixed solvent of dichloromethane and DMSO and sufficiently dissolving until the solution became transparent to the naked eye. A 0.1% polyvinyl alcohol (viscosity: 4.8 to 5.8 mPa s) aqueous solution was used as a continuous phase, and a microsphere was prepared by connecting the continuous phase to an emulsifying device equipped with a porous membrane with a diameter of 40 µm and injecting the prepared dispersed phase at the same time, and the microsphere suspension was put in a preparation container and stirred at a speed of 200 rpm.

The temperature of the membrane emulsifying device and the preparation container was maintained at 25°C, and after the injection of the dispersed phase was completed, the suspension in the preparation vessel was stirred at the speed of 200 rpm while maintaining at the temperature of 25°C for 60 minutes. Then, an organic solvent was removed while maintaining at a temperature of 45°C for 3 hours. When the organic solvent was removed, the temperature of the microsphere suspension was lowered to 25°C. The microsphere suspension was repeatedly washed with ultrapure water to remove residual polyvinyl alcohol, and the microspheres were freeze-dried.

The biocompatible polymer used as the dispersed phase was PLA polymer, and Purasorb PDL04A ((IV=0.35-0.45 dL/g; Manufacturer: Corbion, Germany), Purasorb PDL02A ((IV=0.16-0.24 dL/g; Manufacturer: Corbion, Germany), Resomer R 205S (IV=0.55-0.75 dL/g; Manufacturer: Evonik, Germany), Resomer R 203H (IV=0.25-0.35 dL/g; Manufacturer: Evonik, Germany), Resomer R 202H (IV=0.16-0.24 dL/g; Manufacturer: Evonik, Germany), and Resomer R 205S (IV=0.55-0.75 dL/g; Manufacturer: Evonik, Germany) were used alone or in mixtures.

Types and used amount of polymer, used amount of drug, used amount of pamoic acid, solvents used, used amount of a solvent for the dispersed phase, and used amount of the continuous phase which are used in the present preparation example are as shown in Table 1 below.

**[Table 1]**

| **Category** | **Types of polym ers** | **Used amount of drug (g)** | **Used amoun t of pamoic acid (g)** | **Ratio of Drug:P amoic acid** | **Used amount of polymer (g)** | **Solvent (DCM:D MSO)** | **Used amount of a solvent for the disperse d phase (g)** | **Used amount** of **continuou s phase (ml)** |
|---|---|---|---|---|---|---|---|---|
| Example 1 | PDL04 A | 2 | 0.2 | 1:0.1 | 2.8 | 1:0 | 15.9 | 3,000 |
| Example 2 | PDL04 A | 0.8 | 0.082 | 1:0.1 | 1.118 | 1:0 | 6.3 | 3,000 |
| Example 3 | PDL04 A | 0.8 | 0.082 | 1:0.1 | 1.118 | 4.35:1 | 6.3 | 3,000 |
| Example | PDL04 | 0.8 | 0.085 | 1:0.1 | 1.115 | 1.07:1 | 6.3 | 3,000 |
| 4 | A | | | | | | | |
| Example 5 | PDL04 A | 2.5 | 0.256 | 1:0.1 | 2.244 | 1:0 | 12.7 | 3,000 |
| Example 6 | PDL04 A | 2.5 | 0.256 | 1:0.1 | 2.244 | 1:0 | 12.7 | 3,000 |
| Example 7 | PDL04 A | 2.5 | 0.256 | 1:0.1 | 2.244 | 1:0 | 12.7 | 3,000 |
| Example 8 | R205S | 3 | 0.461 | 1:0.15 | 1.539 | 1:0 | 11.3 | 3,000 |
| Example 9 | R203H | 2.25 | 0.28 | 1:0.125 | 2.47 | 1:0 | 14 | 2,000 |
| Example 10 | PDL04 A | 2.5 | 0.256 | 1:0.1 | 2.24 | 1:0 | 12.7 | 3,000 |
| Example 11 | PLD06 | 1.95 | 0.349 | 1:0.175 | 0.7 | 1:0 | 9.3 | 3,000 |
| Example 12 | PDL04 A | 2.25 | 0.184 | 1:0.08 | 2.566 | 1:0 | 14.5 | 3,000 |
| Comparat ive Example 1 | PDL04 A | 0.8 | 0.091 | 1:0.1 | 1.109 | 1.07:1 | 6.3 | 3,000 |

### Preparation Example 2: Preparation of a sustained-release microsphere containing rivastigmine

A dispersed phase was prepared by mixing a biocompatible polymer, rivastigmine (Rivastigmine base), pamoic acid, and either DCM alone or a co-solvent of DCM and DMSO. A 0.5% (w/v) polyvinyl alcohol aqueous solution was used as a continuous phase, and a dispersed phase was injected and stirred into the continuous phase using a homogenizer to prepare a microsphere.

The temperature of a preparation container was maintained at 25°C, and after the injection of the dispersed phase was completed, the temperature of the microsphere suspension was heated to 45°C and maintained for 3 hours to remove an organic solvent. When the organic solvent was removed, the temperature of the microsphere suspension was lowered to 25°C. The microsphere suspension was repeatedly washed for several times with distilled water, and then the microspheres were recovered and dried.

The biocompatible polymer used as the dispersed phase was PLGA polymer, and Resomer RG 503H (IV=0.32-0.44 dL/g; manufacturer: Evonik, Germany), Resomer RG 653H (IV=0.32-0.44 dL/g; manufacturer: Evonik, Germany), Resomer RG 753H (IV=0.32-0.44 dL/g; manufacturer: Evonik, Germany), Resomer RG 753S (IV=0.32-0.44 dL/g; manufacturer: Evonik, Germany) and Resomer RG 858S (IV=1.3-1.7 dL/g; manufacturer: Evonik, Germany), and Resomer R 203H (IV=0.25-0.35 dL/g; manufacturer: Evonik, Germany) and Resomer R 203S (IV=0.25-0.35 dL/g; manufacturer: Evonik, Germany) as PLA polymer were used alone or in mixtures.

Types and used amount of polymers, used amounts of drug, used amount of pamoic acid, solvents used, used amount of a solvent for the dispersed phase, and used amount of the continuous phase which are used in the present preparation example are as shown in Table 2 below.

**[Table 2]**

| | **Microsp here polymer** | **Used amount of drug (g)** | **Used amount of pamoic acid (g)** | **Ratio of Drug:Pa moic acid** | **Used amount of polymer (g)** | **Solvent (DCM:D MSO)** | **Used amount of a solvent for the disperse d phase (g)** | **Used amount of continuo us phase (ml)** |
|---|---|---|---|---|---|---|---|---|
| Example 13 | 753H+85 8S (1:1) | 1.11 | 0.86 | 1 : 0.50 | 2.48 | 1:0 | 22.5 | 4,500 |
| Example | 753H+85 | 1.11 | 0.86 | 1 : 0.50 | 2.48 | 9:1 | 25 | 4,500 |
| 14 | 8S (1:1) | | | | | | | |
| Example 15 | 753H+85 8S (1:1) | 1.11 | 0.86 | 1 : 0.50 | 2.48 | 9:1 | 25 | 4,500 |
| Example 16 | 753S | 1.57 | 1.22 | 1 : 0.50 | 3.5 | 1:0 | 23.33 | 3,500 |
| Example 17 | 755S | 2.25 | 1.74 | 1 : 0.50 | 5 | 1:0 | 33.33 | 4,700 |
| Example 18 | 503H+20 3H (1:2) | 2.7 | 2.09 | 1 : 0.50 | 6 | 1:0 | 30 | 4,500 |
| Example 19 | 653H+20 3H (1:2) | 3.85 | 2.99 | 1 : 0.50 | 6 | 1:0 | 30 | 4,500 |
| Example 10 | 653H+20 3H (1:3) | 2.16 | 1.67 | 1 : 0.50 | 4.8 | 1:0 | 24 | 3,600 |
| Example 21 | 7535+20 3S (1:1) | 2.16 | 1.67 | 1 : 0.50 | 4.8 | 1:0 | 24 | 3,600 |
| Example 22 | 753H+20 3H (1:2) | 2.1 | 1.63 | 1 : 0.50 | 4.67 | 1:0 | 23.33 | 3,500 |
| Example 23 | 7535+20 3S (1:4) | 1.98 | 1.54 | 1 : 0.50 | 4.41 | 1:0 | 22.06 | 4,500 |

### Preparation Example 3: Preparation of a sustained-release microsphere containing leuprorelin acetate

A dispersed phase was prepared by mixing a biocompatible polymer, leuprorelin acetate (Leuprorelin acetate), pamoic acid, and a co-solvent of DCM and methyl alcohol. A 1.0% (w/v) polyvinyl alcohol aqueous solution was used as a continuous phase, and a microsphere was prepared by connecting the continuous phase to an emulsifying device equipped with a porous membrane with a diameter of 20 µm and injecting the prepared dispersed phase at the same time, and the microsphere suspension was put in a preparation container and stirred at a speed of 200 rpm.

The temperature of a preparation container was maintained at 15°C for 24 hours, and 2,500 mL of 40.0% (w/v) ethyl alcohol was added to the dispersed phase to remove an organic solvent. A dispersed phase exchange process was performed two times with 5,000 mL of 0.5% (w/v) polyvinyl alcohol and 20.0% (w/v) ethyl alcohol aqueous solution. After the organic solvent was removed, the microsphere suspension was repeatedly washed for several times with distilled water, and then the microspheres were recovered and dried.

The biocompatible polymer used as the dispersed phase was PLA polymer, and Resomer R202H (IV=0.16-0.24 dL/g; manufacturer: Evonik, Germany) was used alone.

Types and used amount of polymers, used amount of drugs, used amount of pamoic acid, solvents used, used amount of a solvent for the dispersed phase, and used amount of the continuous phase which are in the present preparation example are as shown in Table 3 below.

**[Table 3]**

| **Categ ory** | **Types of polymer s** | **Used amount of pamoic acid (g)** | **Used amount of pamoic acid (g)** | **Ratio of Drug:Pamo ic acid** | **Used amou nt of polym er (g)** | **Solvent (DCM:MeOH)** | **Used amount of a solvent for the disperse d phase (g)** | **Used amou nt of contin uous phase (ml)** |
|---|---|---|---|---|---|---|---|---|
| Examp le 24 | R202H | 0.888 | 0.013 | 1:0.05 | 3.174 | 1.3:1 | 11.431 | 5,000 |
| Examp le 25 | R202H | 0.888 | 0.026 | 1:0.1 | 3.174 | 1.3:1 | 11.431 | 5,000 |

### Preparation Example 4: Preparation of a sustained-release microsphere containing entecavir

A dispersed phase was prepared by mixing Resomer RG 858S (manufacturer: Evonik, Germany) and Resomer R 203H (manufacturer: Evonik, Germany), which are biocompatible polymers, at a ratio of 7:3 or 5:5, and adding dichloromethane (manufacturer: J.T Baker, USA) to the polymer mixture, followed by stirring until sufficiently dissolved. Subsequently, dimethyl sulfoxide was added to entecavir (manufacturer: Kyungbo Pharmaceutical, Korea) and pamoic acid (manufacturer: J.T Baker, USA), stirred until dissolved, and the two solutions were mixed for use. A 0.5% polyvinyl alcohol aqueous solution with 5% (w/w) NaCl added was used as a continuous phase. After installing a high-speed stirrer in the continuous phase, stirring was performed at 3,000 rpm and the dispersed phase was injected at the same time to prepare an emulsion in which biodegradable polymer containing entecavir were dispersed, and the microspheres were recovered using a sieve immediately after preparation and was washed sufficiently. Thereafter, the suspension was transferred back to a preparation container to stir, and remaining organic solvent was removed while maintaining at 45°C for 3 hours. Then, the temperature of the microsphere suspension was lowered to room temperature, the microspheres were filtered, treated for removing residual polyvinyl alcohol with triple-distilled water, and then freeze-dried.

Types and used amount of polymers, used amount of drugs, used amount of pamoic acid, solvents used, used amount of a solvent for the dispersed phase, and used amount of the continuous phase which are used in the present preparation example are as shown in Table 4 below.

**[Table 4]**

| **Catego ry** | **Types of polymer s** | **Used amount of drug (g)** | **Used amount of pamoic acid (g)** | **Ratio of Drug:Pa moic acid** | **Used amount of polymer (g)** | **Solvent (DCM:D MSO)** | **Used amount of a solvent for the disperse d phase (g)** | **Used amount of continu ous phase (ml)** |
|---|---|---|---|---|---|---|---|---|
| Exampl e 26 | RG858S: 203H(7:3 ) | 0.30 | 0.042 | 1:0.1 | 0.658 | 1:1.13 | 7.9 | 1,000 |
| Exampl e 27 | RG858S: 202H(5:5 ) | 0.30 | 0.021 | 1:0.05 | 0.679 | 1:1.29 | 8.5 | 1,000 |
| Compar ative Exampl e 2 | RG858S: 203H(7:3 ) | 0.30 | 0.042 | 1:0.1 | 0.658 | 1:1.13 | 7.9 | 1,000 |

### Preparation Example 5: Preparation of a sustained-release microsphere containing octreotide

A dispersed phase was prepared by adding dichloromethane (manufacturer: J.T Baker, USA) to a biocompatible polymer and stirring until sufficiently dissolved. Thereafter, dimethyl sulfoxide (manufacturer: J.T Baker, USA) was added to octreotide (manufacturer: Hemmo, India) and pamoic acid, stirred until dissolved, and then the two solutions were mixed for use. A 0.1% polyvinyl alcohol aqueous solution with 5% (w/w) NaCl added was used as a continuous phase. Microspheres were prepared by connecting 2,000 mL of the continuous phase to an emulsifying device equipped with a porous membrane with a diameter of 30 µm and injecting the prepared dispersed phase at the same time, and the suspension was stirred at 200 rpm. Then, remaining organic solvent was removed while maintaining at 40°C for 3 hours. When the removal was completed, the temperature of a microsphere suspension was lowered to room temperature, the microspheres were filtered, treated for removing residual polyvinyl alcohol with triple-distilled water, and then freeze-dried.

The biocompatible polymer used as the dispersed phase was RG 753H (manufacturer: Evonik, Germany, viscosity: 0.32-0.44 dL/g), PDLG 7504A (manufacturer: Corbion, Netherlands, viscosity: 0.39-0.48 dL/g), and RG 853H (manufacturer: Evonik, Germany, viscosity: 0.44 dL/g) used alone or in mixtures.

Types and used amount of polymers, used amount of drugs, used amount of pamoic acid, solvents used, used amount of a solvent for the dispersed phase, and used amount of the continuous phase which are used in in the present preparation example are as shown in Table 5 below.

**[Table 5]**

| **Categor y** | **Types of polymer s** | **Used amount of drug (g)** | **Used amount of pamoic acid (g)** | **Ratio of Drug:Pa moic acid** | **Used amount of polymer (g)** | **Solvent (DCM:D MSO)** | **Used amount of a solvent for the disperse d phase (g)** | **Used amount of continuo us phase (ml)** |
|---|---|---|---|---|---|---|---|---|
| Example 28 | 753H | 0.341 | 0.029 | 1:0.25 | 0.631 | 1:1.36 | 5.3 | 2,000 |
| Example 29 | 7504A | 0.348 | 0.114 | 1:1 | 0.537 | 1:0.40 | 14.25 | 5,000 |
| Example 30 | 7504A | 0.290 | 0.095 | 1:1 | 0.614 | 1:0.33 | 15.56 | 2,000 |
| Example 31 | 753H:853 H=1:1 | 0.284 | 0.048 | 1:0.5 | 0.669 | 1:0.29 | 7.76 | 2,000 |
| Compar ative Example 3 | 7504A | 0.348 | N/A | N/A | 0.652 | 1:0.25 | 7.37 | 2,000 |

### Experimental Example 1. Measurement of drug content of the sustained-release microsphere containing donepezil

In order to measure a drug content of the microsphere prepared in Preparation Example 1, 10 mg of the microsphere were completely dissolved in dimethyl sulfoxide and then diluted with a mobile phase. 20 µL of the diluted solution was injected into HPLC and measured at a detection wavelength of 271 nm. A column used in the present measurement was Inertsil ODS-3, 5 µm, 4.6x150 mm, and the mobile phase was a mixture of phosphate buffer (pH5.0) and acetonitrile at a ratio of 60:40 (v/v). The measured amount of encapsulated drug is shown in Table 6.

### Experimental Example 2. Measurement of drug content of a rivastigmine sustained-release microsphere

In order to measure a drug content of the microsphere prepared in Preparation Example 2, 45 mg of the microsphere was completely dissolved in 25 mL of dimethyl sulfoxide and then diluted with a mobile phase. 20µL of the diluted solution was injected into HPLC and measured at a detection wavelength of 214 nm. A column used in the present measurement was Inertsil ODS-3, 5 µm, 4.6x250mm, and the mobile phase was a mixture of phosphate buffer (pH 7.0) and acetonitrile at a ratio of 77:23 (v/v). The measured amount of encapsulated drug is shown in Table 6.

### Experimental Example 3. Measurement of drug content of the acetate sustained-release microsphere containing leuprolide

In order to measure a drug content of the microsphere prepared in Preparation Example 3, 10 mg of the microsphere was completely dissolved in 2.5 mL of acetonitrile, and then 7.5 mL of 0.1% (w/w) trifluoroacetic acid aqueous solution was added to extract the microspheres. A filtrate using a 0.45 µm filter was used as a test solution. 20 µL of the test solution was injected into HPLC and measured at a detection wavelength of 280 nm. A column used in the present measurement was Inertsil ODS-3, 5 µm, 4.6x150 mm, and the mobile phase was a mixture of acetonitrile containing 0.1% (w/w) trifluoroacetic acid and a 0.1% (w/w) trifluoroacetic acid aqueous solution at a ratio of 25:75 (v/v). The measured amount of encapsulated drug is shown in Table 6.

### Experimental Example 4. Measurement of drug content of the sustained-release microsphere containing entecavir

In order to measure a drug content of the microsphere prepared in Preparation Example 4, 10 mg of the microsphere was completely dissolved in dimethyl sulfoxide and then diluted again with dimethyl sulfoxide. 5 µL of the diluted solution was injected into HPLC and measured at a detection wavelength of 214 nm. A column used in the present measurement was Inertsil ODS-3, 5 µm, 4.6x150 mm, and the mobile phase was a mixture of acetonitrile containing 0.1% (w/w) trifluoroacetic acid and a 0.1% (w/w) trifluoroacetic acid aqueous solution at a ratio of 91.5:8.5 (v/v). The measured amount of encapsulated drug is shown in Table 6.

### Experimental Example 5. Measurement of drug content in the sustained-release microsphere containing octreotide

In order to measure a drug content of the microsphere in Preparation Example 5, 10 mg of the microsphere was completely dissolved in dimethyl sulfoxide and then diluted again with dimethyl sulfoxide. 10 µL of the diluted solution was injected into HPLC and measured at a detection wavelength of 254 nm. A column used in the present measurement was Inertsil ODS-3, 5 µm, 4.6x150 mm, and the mobile phase was a mixture of acetonitrile containing 0.1% (w/w) trifluoroacetic acid and a 0.1% (w/w) trifluoroacetic acid aqueous solution at a ratio of 80:20 (v/v). The measured amount of encapsulated drug is shown in Table 6.

### Experimental Example 6. Particle size analysis of sustained-release microspheres

In order to quantitatively measure an average particle size, distribution, and uniformity of the microsphere, tests were conducted using the laser diffraction method.

The particle size measurement results of the sustained-release microsphere prepared in Examples and Comparative Examples are shown in Table 6 below.

**[Table 6]**

| **Category** | **Drug content ((w/w)%)** | **Drug encapsulation efficiency (%)** | **Microsphere size (D50, µm)** |
|---|---|---|---|
| Example 1 | 35.3 | 88.3 | 49.6 |
| Example 2 | 39.4 | 98.6 | 52.1 |
| Example 3 | 35.4 | 88.5 | 63.2 |
| Example 4 | 38.1 | 95.3 | 62.2 |
| Example 5 | 48.3 | 96.6 | 47.2 |
| Example 6 | 48.3 | 96.6 | 51.2 |
| Example 7 | 47.6 | 95.2 | 51.9 |
| Example 8 | 57.6 | 91.7 | 48.6 |
| Example 9 | 42.4 | 94.3 | 58.9 |
| Example 10 | 45.1 | 90.1 | 46.6 |
| Example 11 | 59.0 | 90.7 | 47.6 |
| Example 12 | 41.27 | 91.7 | 51.02 |
| Example 13 | 24.2 | 96.8 | 39.4 |
| Example 14 | 23.6 | 94.4 | 35.7 |
| Example 15 | 22.5 | 90.0 | 39.0 |
| Example 16 | 19.8 | 79.3 | 42.9 |
| Example 17 | 21.0 | 84.0 | 56.1 |
| Example 18 | 21.5 | 86.0 | 50.7 |
| Example 19 | 25.6 | 85.4 | 50.7 |
| Example 20 | 21.0 | 83.8 | 47.7 |
| Example 21 | 20.1 | 80.3 | 47.4 |
| Example 22 | 20.5 | 82.2 | 47.6 |
| Example 23 | 20.2 | 80.9 | 66.9 |
| Example 24 | 17.0 | 84.9 | 40.0 |
| Example 25 | 18.4 | 92.1 | 24.31 |
| Example 26 | 27.7 | 92.3 | 68.1 |
| Example 27 | 25.4 | 84.6 | 66.6 |
| Comparativ e Example 2 | 10.8 | 36.0 | 64.4 |
| Example 28 | 25.3 | 84.2 | 50.4 |
| Example 29 | 10.7 | 35.0 | 42.5 |
| Example 30 | 14.6 | 58.6 | 27.8 |
| Example 31 | 22.1 | 88.3 | 23.5 |
| Comparativ e Example | 24.3 | 80.9 | 27.8 |
| 3 | | | |
| Comparativ e Example 1 | 27.0 | 85.5 | 71.7 |

### Experimental Example 7: Measurement of the content of sodium in a raw material pamoic acid

In order to measure the content of sodium in the pamoic acid used in Examples and Comparative Examples, 300 mg of pamoic acid was mixed with 6 mL of nitric acid aqueous solution mixed with ultrapure water at a ratio of 1:1, and 3 mL of hydrogen peroxide, heated at 100°C or higher, and then acid was added until a gas generated during a dissolution process changed from yellow to white. The obtained sample was weighed, dissolved in ultrapure water, and injected into an inductively coupled plasma optical emission spectrometer (ICP-OES) (Thermo Scientific Co., iCAP 6300 Duo, UK) to be measured at a detection wavelength of 598.5 nm.

The measured amount of sodium is shown in Table 7.

### Experimental Example 8: Measurement of content of sodium in the sustained-release microsphere

In order to measure the content of sodium in the microsphere prepared in Examples and Comparative Examples, the content of sodium was measured in a same manner as in Experimental Example 7 using 300 mg of pamoic acid.

**[Table 7]**

| **Category** | **Content of sodium in pamoate(ppm)** | **Content of sodium in microsphere (ppm)** |
|---|---|---|
| Example 1 | 38 | 14.2 |
| Example 2 | 1,530 | 34.7 |
| Example 3 | 2,600 | 67.4 |
| Example 4 | 33,919 | 601 |
| Example 5 | 456 | 32 |
| Example 6 | 27 | 21 |
| Example 7 | 2,600 | 76 |
| Example 11 | 285 | 26 |
| Example 15 | 2,600 | 29.0 |
| Comparativ e Example 1 | 101,681 | 1,640 |
| Comparativ e Example 2 | 101,681 | 1970 |

### Experimental Example 9: In vitro drug initial release measurement of a donepezil microsphere depending on the content of sodium

The following experiment was performed to verify an initial drug release of a microsphere depending on the content of sodium in pamoic acid used for the microsphere prepared in Preparation Example 1 and the content of sodium in the microsphere. 10 mg of the microsphere was put in an HDPE wide-mouth bottle, filled with 50 mL of release test solution, and stored in a 37°C incubator. After 24 hours, 1 mL of the test solution was taken and centrifuged. The supernatant obtained was analyzed for donepezil content and release rate using HPLC under the same analysis conditions as in Experimental Example 1. A release test solution used for the present measurement was pH 7.4 aqueous solution containing phosphate and sodium azide.

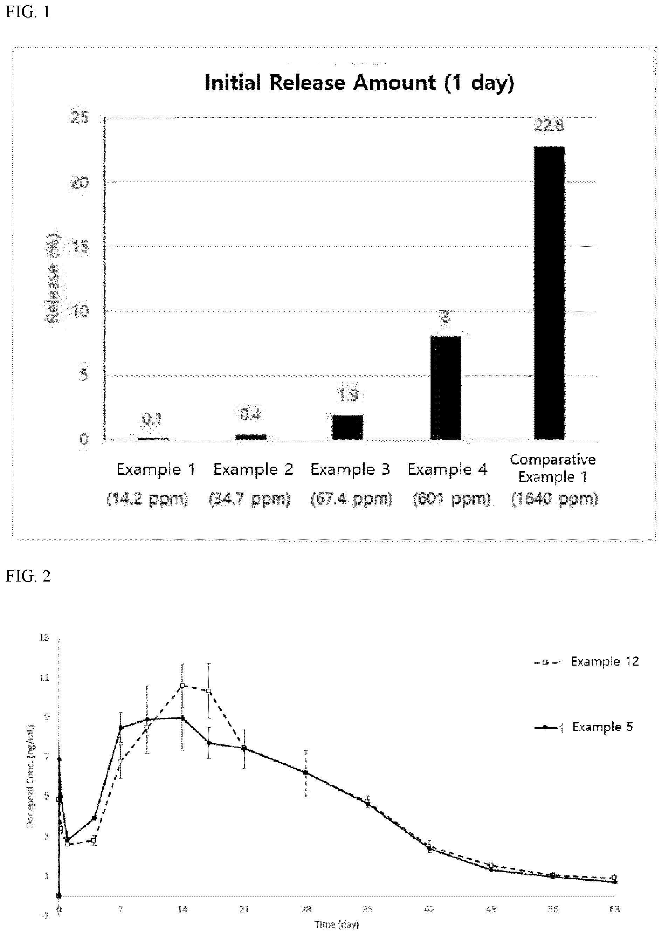

The results are shown in Table 8 and FIG. 1.

### Experimental Example 10: In-vivo drug release measurement depending on the content of sodium in pamoic acid

To evaluate the drug release of a microsphere depending on the content of sodium in pamoic acid used in the microsphere prepared in Examples, the blood concentration of donepezil was measured to confirm pharmacokinetics of the drug after administering donepezil to SD rats once.

The microsphere was measured so as to a donepezil dose be 26.04 mg/head, dispersed in a 0.3 mL suspension, and then intramuscularly injected into SD rats. 0.25 to 0.5 mL of blood was collected for each of pre-scheduled times, and the blood concentration of donepezil was measured using HPLC. The drug concentration (ng/mL) over the time of Examples 5, 6, and 7 is shown in FIG. 2.

### Experimental Example 11: In vitro drug initial release measurement of a rivastigmine microsphere depending on the content of sodium

The following experiment was performed in order to evaluate the initial drug release of a microsphere depending on the content of sodium in pamoic acid used in the microsphere prepared in Preparation Example 2 and the content of sodium in the microsphere.

22 mg of the microsphere was put in an HDPE wide-mouth bottle, filled with 50 mL of a release test solution, and stored in a 37°C incubator. After 24 hours, 1 mL of the test solution was taken and centrifuged. The supernatant obtained was analyzed for a rivastigmine content and release rate using HPLC under the same analysis conditions as in Experimental Example 2. A release test solution used for this measurement was pH 7.4 aqueous solution containing phosphate and sodium azide.

The results are shown in Table 8.

**[Table 8]**

| **Category** | **Content of sodium in raw material pamoic acid (Unit ppm)** | **Content of sodium in microsphere (Unit ppm)** | **Day 1 release amount of drugs (Unit %)** |
|---|---|---|---|
| Example 1 | 38 | 14.2 | 0.1 |
| Example 2 | 1,530 | 34.7 | 0.4 |
| Example 3 | 2,600 | 67.4 | 1.9 |
| Example 4 | 33,919 | 601 | 8 |
| Example 14 | 38 | 8.0 | 0.12 |
| Example 15 | 2,600 | 29.0 | 0.35 |
| Comparati ve Example 1 | 101,681 | 1,640 | 22.80 |
| Comparati ve Example 2 | 101,681 | 1,970 | 15.92 |
| Comparati ve Example 3 | - | - | 40.23 |

As shown in Table 8 above, when the content of sodium contained in the pamoic acid used for the preparation of the microsphere was 10 wt% or more based on the weight of the pamoic acid, and the content of sodium contained in the microsphere was 1,600 ppm or more, an excessive initial release of the drug was observed on day 1. Therefore, the content of sodium in the pamoate used for the microsphere should be below a certain level in the microsphere to ensure that the initial release rate on day 1 remains 15% or less when the microspheres administered, thereby allowing the drug to be gradually released over a long period without excessive initial release of the drug, which is desirable in terms of the drug release.

## Claims

1. A sustained-release microsphere, which is prepared using a drug; pamoic acid; and a biocompatible polymer,
wherein a molar ratio of the drug to the pamoic acid is 1:0.01 to 1:1, and a content of sodium contained in the microsphere is less than 1,600 ppm.

2. A sustained-release microsphere, which is prepared using a drug; pamoic acid; and a biocompatible polymer,
wherein a molar ratio of the drug to the pamoic acid is 1:0.01 to 1:1, and a content of sodium contained in the pamoic acid is 10 wt% or less based on the total weight of the pamoic acid.

3. A sustained-release microsphere comprising a drug; pamoic acid; and a biocompatible polymer, wherein a content of sodium contained in the microsphere is less than 1,600 ppm.

4. The sustained-release microsphere of claim 3, wherein the sustained-release microsphere further contains a pamoate of the drug.

5. The sustained-release microsphere of any one of claims 1 to 3, wherein the sodium is in a form of monosodium or disodium.

6. The sustained-release microsphere of any one of claims 1 to 3, wherein the drug is at least one selected from the group consisting of donepezil, rivastigmine, entecavir, leuprolide, octreotide, and pharmaceutically acceptable salts thereof.

7. The sustained-release microsphere of any one of claims 1 to 3, wherein initial release rate on day 1 of the drug is less than 15%.

8. The sustained-release microsphere of any one of claims 1 to 3, wherein a content of the active ingredient is 10 to 79 wt% based on the total weight of the sustained-release microsphere.

9. The sustained-release microsphere of any one of claims 1 to 3, wherein the drug in the sustained-release microsphere is released for 1 month to 6 months.

10. The sustained-release microsphere of any one of claims 1 to 3, wherein the biocompatible polymer is at least one selected from the group consisting of poly(lactide-co-glycolide), poly(lactide-co-glycolide)glucose, polylactide, polyglycolide, polycaprolactone, and mixtures thereof; and polyglycolide, polylactide and a copolymer of polyglycolide and polylactide.

11. The sustained-release microsphere of any one of claims 1 to 3, wherein the sustained-release microsphere is for injection.

12. A pharmaceutical composition comprising the sustained-release microsphere of any one of claims 1 to 3, wherein the pharmaceutical composition is for prevention, improvement, or treatment of hepatitis B, prostate cancer, breast cancer, endometriosis, uterine fibroid, precocious puberty, acromegaly, gastro·entero·pancreatic endocrine tumor, Alzheimer's disease, or cognitive disorder.

13. The pharmaceutical composition of claim 12, further comprising a pharmaceutically acceptable carrier.

14. The pharmaceutical composition of claim 12, wherein the pharmaceutical composition is an injectable formulation.

15. A preparing method of a sustained-release microsphere according to any one of claims 1 to 3 comprising:
(a) preparing a dispersed phase by dissolving a drug, pamoic acid, and a biocompatible polymer in a solvent for the dispersed phase;
(b) preparing an emulsion by adding the dispersed phase prepared in the step (a) to an aqueous solution containing a surfactant as a continuous phase;
(c) preparing a suspension containing the microsphere by extracting the solvent for the dispersed phase of the dispersed phase in the emulsion prepared in the step (b) into the continuous phase, and evaporating the extracted solvent to form a microsphere; and
(d) preparing the microsphere by recovering the microsphere from the suspension of the step (c),
wherein, a content of sodium contained the pamoic acid is 10 wt% or less based on the weight of the pamoic acid.

16. The preparing method of claim 15, wherein a molar ratio of the drug to the pamoic acid is 1:0.01 to 1:1 based on the free base form of the active ingredient.

17. The preparing method of claim 15, wherein the drug is in the free base form in the step(a).

18. The preparing method of claim 15, wherein the step (a) is performed by adding the pamoic acid to a solution of the drug dissolved in the solvent for the dispersed phase.

19. The preparing method of claim 15, wherein the solvent for the dispersed phase includes dichloromethane, ethyl acetate, ethyl formate, methyl acetate, methyl formate, butyl acetate, n-propyl acetate, isopropyl acetate, n-propyl formate, glycofurol, methyl isopropyl ketone, dimethyl carbonate, or mixed solvents thereof.

20. The preparing method of claim 15, wherein the solvent for the dispersed phase further contains one or more selected from the group consisting of chloroform, acetone, acetonitrile, dimethyl sulfoxide, dimethylformamide, n-methylpyrrolidone, methyl ethyl ketone, acetic acid, methyl alcohol, ethyl alcohol, propyl alcohol, benzyl alcohol, and mixed solvents thereof.

21. The preparing method of claim 15, wherein the biocompatible polymer is at least one selected from the group consisting of poly(lactide-co-glycolide), poly(lactide-co-glycolide)glucose, polylactide, polyglycolide, polycaprolactone, and mixtures thereof; and polyglycolide, polylactide and a copolymer of polyglycolide and polylactide.
